(19) **Europäisches Patentamt / European Patent Office / Office européen des brevets**

(11) **EP 1 259 154 B1**

(12) **EUROPEAN PATENT SPECIFICATION**

(45) Date of publication and mention
of the grant of the patent:
**03.10.2007   Bulletin 2007/40**

(51) Int Cl.:
***A61B 5/00*** *(2006.01)*      ***A61B 5/12*** *(2006.01)*

(21) Application number: **01909137.0**

(86) International application number:
**PCT/US2001/004465**

(22) Date of filing: **13.02.2001**

(87) International publication number:
**WO 2001/060230 (23.08.2001 Gazette 2001/34)**

(54) **AUDIOMETRIC APPARATUS AND ASSOCIATED SCREENING METHOD**

AUDIOMETRIEGERÄT UND DAMIT VERBUNDENES TESTVERFAHREN

APPAREIL AUDIOMETRIQUE ET PROCEDE DE DEPISTAGE ASSOCIE

(84) Designated Contracting States:
**AT BE CH CY DE DK ES FI FR GB GR IE IT LI LU MC NL PT SE TR**

(30) Priority: **14.02.2000   US 182291 P**

(43) Date of publication of application:
**27.11.2002   Bulletin 2002/48**

(73) Proprietor: **Kinderlife Instruments, Inc.**
**Mountain View, CA 94041 (US)**

(72) Inventors:
• **STONE, Robert, T.**
**Sunnyvale, CA 94087 (US)**

• **HERSCHER, Bret, A.**
**Cupertino, CA 95014 (US)**

(74) Representative: **Exell, Jonathan Mark**
**Williams Powell**
**Morley House**
**26-30 Holborn Viaduct**
**London EC1A 2BP (GB)**

(56) References cited:
| | |
|---|---|
| WO-A-97/23117 | WO-A-99/40533 |
| US-A- 4 809 708 | US-A- 4 913 160 |
| US-A- 5 143 081 | US-A- 5 282 475 |
| US-A- 5 546 956 | US-A- 5 885 225 |

**Description**

**FIELD OF THE INVENTION**

The present invention relates generally to the field of audiometric hearing screening devices and associated screening methods. More particularly, the invention relates to an audiometric apparatus and auditory screening method that employs true random stimuli sequences and sampling frequencies.

**BACKGROUND OF THE INVENTION**

Language acquisition in infants requires a critical period of hearing capacity, which spans the frequency range of human speech. The critical period extends from birth to about two to three years of age, when infants typically begin to talk with some level of proficiency.

It has however been reported that approximately three to five percent of newborn infants suffer from some degree of hearing impairment. These impairments can be devastating to the social, emotional and intellectual development of the affected infants. Early identification of hearing impairments in infants allows for early intervention to minimize significant speech and language deficiencies.

Infants are however usually unable or unwilling to participate in known behavioral auditory examinations. Moreover, delaying auditory screening until infants can verbally respond is often too late for hearing impaired infants and in many instances, results in long term problems.

Federal, state and private agencies have attempted to implement universal auditory screening of infants for over twenty years. A major impediment to the implementation of universal auditory screening of infants has been the cost and complexity associated with the tests. Current infant screening tests are time consuming and require expensive devices and trained specialists to conduct the tests and interpret results. As such, universal auditory screening of infants is presently economically infeasible.

Various entities have developed audiometric devices, which may be usable for screening an infant's hearing. These existing devices generally fall into one of two categories. Devices in the first category are configured to elicit auditory evoked potentials (AEP's), which are electrical responses of cells within the auditory pathway of the brain to an acoustic stimulus. Such devices typically utilize the non-invasive auditory brainstem response (ABR) test for auditory screening of infants. An earphone provides an acoustic stimulus, specifically a brief click or toneburst, to the subject's ear. Electrodes attached to the subject's scalp receive auditory evoked potentials (i.e., response signal(s)) from the scalp, which are recorded as an electroencephalogram waveform. Analysis of these brainwave patterns are used to determine if the auditory system is functioning normally.

Devices in the second category utilize the evoked otoacoustic emission (OAE) test for auditory screening. An earphone provides a brief acoustic stimulus to the subject's ear. A microphone disposed in the subject's ear adjacent the earphone receives an OAE from the ear, which is recorded as an acoustic signal. Analysis of the OAE waveform provides an indication of the functional integrity of the middle and inner ear, which together comprise the auditory periphery.

The noted audiometric screening devices have numerous drawbacks and disadvantages. A major drawback is that response signals are susceptible to undesirable artifact components and/or noise, which can emanate from the device itself or the infant (e.g.; swallowing, grinding of teeth).

As will be appreciated by one having ordinary skill in the art, the evoked potentials (or response signals) are relatively small in magnitude (< 1 microvolt) compared to general EEG activity (i.e., neurological electrical noise) levels. Thus, techniques such as signal averaging and the deployment of "pseudo-random" sequences (i.e., pseudo-random pulse trains) have been employed for diagnostic evaluations to enhance the signal-to-noise ratio, and, hence, separate the response signal(s) from the background noise.

The technique of averaging response signals across multiple trials to estimate the response signal - evoked potentials-to a stimulus is based on two assumptions: (1) that the signal does not change across the trials and (2) that the background electrical activity has no time-locked relationship to the stimulus and is a random process with a mean potential of zero.

Even if one were to accept the questionable assumption that the underlying signal is homogenous across trials, the average signal remains only an estimate of the base (i.e., true) signal. Further, the average signal would still include residual EEG noise, as well as the base signal.

Techniques employing pseudo-random sequences, such as Maximum Length Sequences or M-pulse Sequences, are similarly based on the assumption that the background electrical activity has no time-locked relationship to the stimulus. Notwithstanding this base assumption, although the stimuli or pulses arc randomly spaced, the spacing is typically in multiples of a time interval.

Accordingly, any multiple of the stimulus (e.g., 37 clicks/sec.) that is in the environment will corrupt the base signal and produce a "synchronous artefact". Similarly, any multiple of the sample frequency will produce a "sampling artefact".

It is therefore an object of the present invention to provide an audiometric apparatus and auditory screening method that provides rapid, low-cost, comprehensive, non-invasive screening of a person's hearing.

It is another object of the present invention to provide an audiometric apparatus and auditory screening method that employs true random stimuli sequences that substantially reduce or eliminate synchronous artefacts.

It is yet another object of the present invention to provide an audiometric apparatus and auditory screening method that employs true random sampling frequencies that substantially reduce or eliminate sampling artefacts.

United States Patent Number 4,809,708 presents a method and apparatus for real bar measurements. In the presented method a probe tube is inserted into the car canal of a patient so that the probe tip is spaced well away from the eardrum. A broad band sound signal is provided to the patient's ear, with the probe tip being positioned at different positions in the ear canal, and the frequency response of detected sound at the probe tip for the various positions is determined.

## SUMMARY OF THE INVENTION

In accordance with the above objects and those that will be mentioned and will become apparent below, the audiometric apparatus in accordance with this invention comprises stimulus generating means for transmitting at least one true random stimulus sequence to a subject's inner ear and detection means for detecting the response signal returned from the subject's inner car in response to the stimulus sequence.

The auditory screening method in accordance with this invention comprises the steps of (i) presenting at least one true random stimulus sequence to said subject's inner ear and (ii) detecting the response signal returned from the subject's inner ear in response to the stimulus sequence.

In an additional embodiment of the invention, the method for testing hearing of a subject comprises the steps of (i) presenting at least one stimulus sequence to said subject's inner ear, (ii) detecting the response signal returned from the subject's inner ear in response to the stimulus sequence, the response signal having at least one waveform, (iii) sampling the response signal waveform by applying a plurality of true random frequencies to the response signal, the sampling providing at least a first set of response signal data, (iv) recording the first set of response signal data, and (v) reconstructing the response signal waveform from the first set of response signal data.

## BRIEF DESCRIPTION OF THE DRAWINGS

Further features and advantages will become apparent from the following and more particular description of the preferred embodiments of the invention, as illustrated in the accompanying drawings, and in which like referenced characters generally refer to the same parts or elements throughout the views, and in which:

FIGURE 1 is a schematic illustration of one embodiment of the audiometric apparatus according to the invention;

FIGURE 2 is a graphical illustration of true random stimulus sequences according to the invention;

FIGURE 3 is a graphical illustration of an exemplary EEG response signal;

FIGURE 4 is a schematic illustration of a pseudo-random sampler;

FIGURE 5 is a schematic illustration of an additional embodiment of the audiometric apparatus according to the invention;

FIGURE 6 is a block diagram of the sampling means according to the invention;

FIGURE 7 is a block diagram of one embodiment of the noise (or signal) generator according to the invention;

FIGURE 8 is a graphical illustration of the comparator output according to the invention; and

FIGURE 9 is a schematic illustration of the averager according to the invention;

## DESCRIPTION OF THE PREFERRED EMBODIMENT

As discussed in detail below, the present invention substantially reduces the disadvantages and drawbacks of the noted prior art devices and techniques. According to one embodiment of the invention, the audiometric apparatus employs at least one "true random" sequence - varying stimuli frequency and rate - to substantially reduce or eliminate "synchronous artifacts". In an additional embodiment of the invention, the apparatus includes "true random" sampling means to eliminate "sampling artifacts".

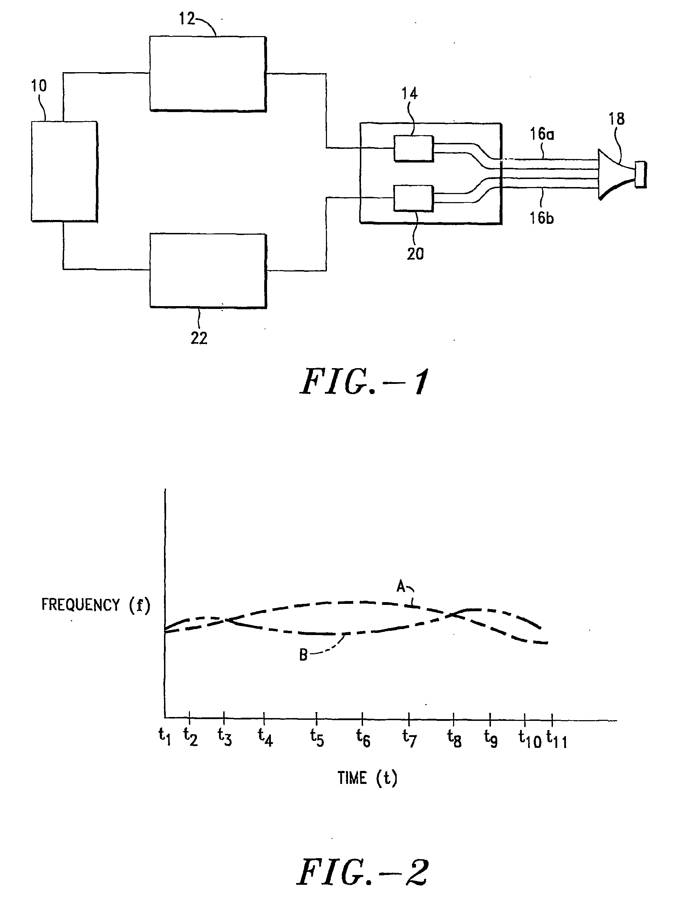

Referring first to Figure 1, there is shown a schematic illustration of one embodiment of the invention. The apparatus includes an analyzer 10, which may be a computer, microprocessor or other analytical apparatus employed to perform the averaging and other analytical and control functions.

As illustrated in Figure 1, the analyzer 10 is in communication with and controls the stimulus generating means 12 of the invention. According to the invention, the stimulus generating means 12 may be a separate component or integral with the analyzer 10.

The stimulus provided by the stimulus generating means 12 is then transmitted to a small transducer 14 which feeds a sound wave into the ear canal of the person being tested, via input line 16a and a small earpiece 18 designed to fit into the ear canal. The sound reflected from the inner ear (i.e., response signal) is transmitted to a small microphone 20 via the earpiece 18 and output line 16b.

The microphone 20 conveys the response signal to signal conditioning equipment 22, which typically includes pre-amplifiers, filters and amplifiers. The output (i.e., detected and conditioned response signal(s)) from the signal conditioning equipment 22 is then transmitted to the analyzer 10 for selective analysis.

As indicated above, a key feature of the apparatus illustrated in Fig. 1 is that the stimulus provided by the stimulus generating means 12 comprises "true random" sequences. By the term "true random", it is meant to mean substantially devoid of a definitive pattern or relationship with time.

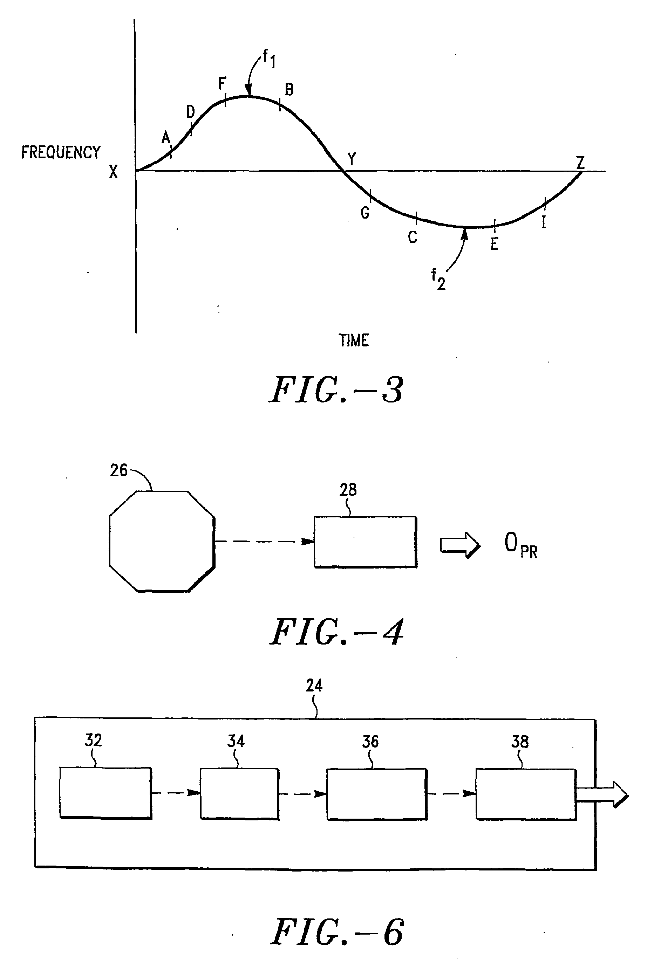

Referring now to Figure 2, there is shown a graphical illustration of "true random" sequences according to the invention. Curve A illustrates (i) a first sampling comprising a gradually increasing stimulus rate (e.g., 36.5 clicks/sec. to 38.5/ sec.) while gradually increasing the time between the stimuli and (i) a second sampling comprising a gradually decreasing stimulus rate while gradually increasing the time between the stimuli. Curve B illustrates a two cycle frequency deviation over gradually increasing then decreasing time intervals.

Curves A and B are merely illustrations of two forms of stimuli variation according to the invention. As will be appreciated by one having ordinary skill in the art, the "true random" sequences of the invention comprise and include numerous variations of stimulus rate and time sequences.

According to the invention, the variation in stimulus rate is typically in the range of +/-10 to 50 %, preferably in the range of +/-30 to 50 %. The noted range would not be deemed biologically significant, but is significant with regards to signal frequencies.

As will be further appreciated by one having skill in the art , if a synchronous signal (i.e., artifact) is included in the base signal, it's effects will be:

$$\frac{1}{\text{No. of variations in process}}$$

and it becomes asynchronous with regard to the stimulus rate. Accordingly, since signals in the environment typically exhibit a constant frequency, the probability of experiencing a frequency that would sweep at the same rate as the "true random" stimuli of the invention is virtually zero.

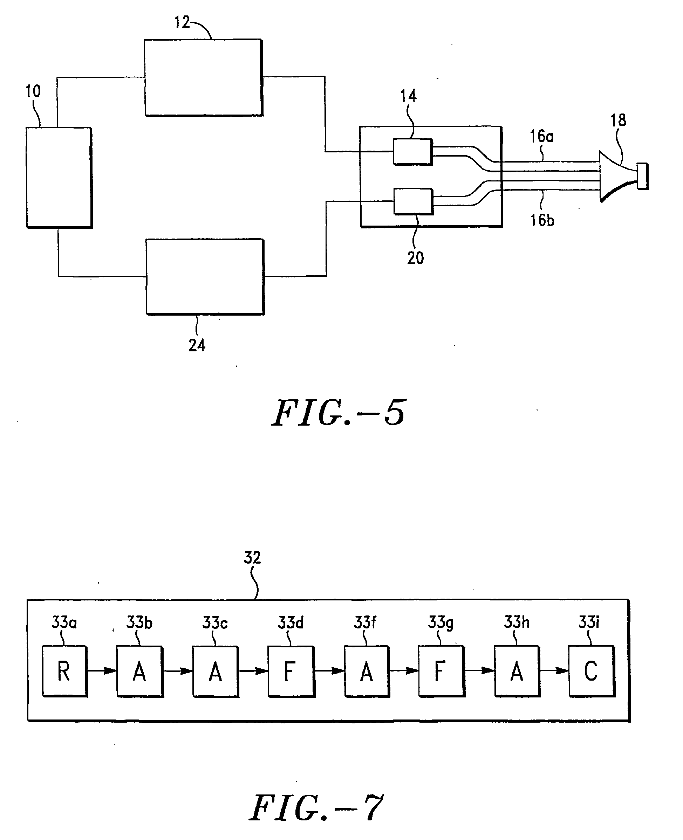

Referring now to Figure 3, there is shown an exemplary portion of an EEG response signal. If the frequency of the signal is known and one wishes to obtain an accurate representation of the frequency by digital sampling, pursuant to well known sampling theorems, one must sample at a rate that is greater than 2x the frequency of the base signal.

If, however, the frequency is unknown, digital sampling cannot be employed. In those instances, a conventional approach is to filter the signal to eliminate sample components that are greater than one half the frequency of the sampling signal. Although the noted approach will, in many instances, provide an accurate assessment of the frequency of the base signal, the approach will typically <u>not</u> provide an accurate indication of the signal's waveform. By way of example, assuming one were able to sample at points X and Y at a rate of 5 KHz, the noted sampling sequence would indicate a waveform having a frequency equal to zero (see Figure 3). Sampling at points X and Y, would also provide little, if any, information on the waveform of the signal. Even if one were to sample at points X, Y, and Z at a rate of 20 KHz, it is still unlikely that a representative waveform would be generated.

If, however, one were able to "randomly sample" at points A-I in the following sampling sequence: (i) points A, B and C (ii) points D and E and (iii) points F, G and I, two advantageous results are achieved. First, it is virtually impossible for extraneous artifacts to be introduced into the signal. Second, a good representation of the signal's waveform can be provided without high sampling rates, which typically require extensive computing power.

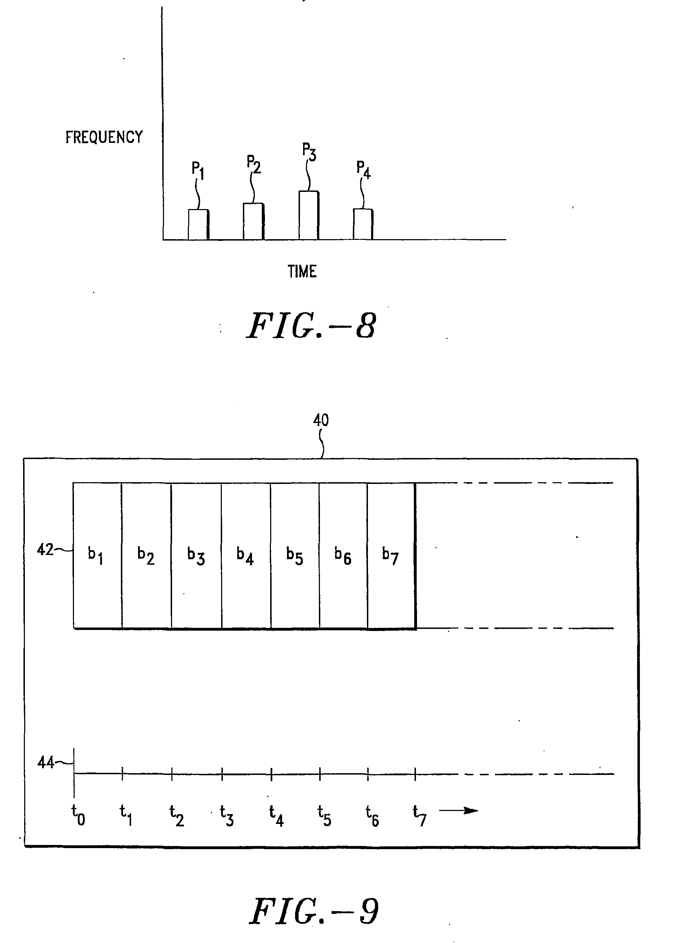

A major drawback of conventional " random samplers" is, however, that they typically employ a fixed clock (i.e., pseudo-random digital samplers). Referring to Figure 4, there is shown a schematic illustration of a conventional pseudo-random sampler having a crystal 26 of known "fixed" frequency (e.g., 20 KHz) and a pseudo-random processor 28.

As will be appreciated by one having ordinary skill in the art, the output signals ($O_{pr}$) from the pseudo-random processor 28 would all be at some sub-harmonic or multiple of the 20 KHz signal. Thus, since all of the digital pseudo-random samplers contain the base clock frequencies, the signals produced therefrom would be susceptible to specific artifacts.

In contrast to a conventional random sampler, the present invention provides and, hence, employs continuously "true random" sampling frequencies. According to the invention, the "true random" frequencies are provided by the sampling means 24 of the invention. As illustrated in Fig. 5, the sampling means 24 can be substituted for the conventional signal processing equipment 22. As discussed in detail below, the apparatus shown in Fig. 5, which employs "true random" stimuli sequences and "true random" sampling frequencies provides an accurate reconstructed waveform that is virtually devoid of extraneous artifacts (i.e., noise).

Referring now to Figure 6, there is shown a simple block diagram of the sampling means 24 of the invention that is preferably employed to produce continuously "true random" frequencies. According to the invention, a true noise generator 32 is preferably employed to provide an initial, random signal sequence.

Referring now to Figure 7, there is shown a block diagram of one embodiment of the noise (or signal) generator 32. The noise generator 32 preferably includes a resistor 33a having a resistance in the range of 100,000 to 500,000 Ohms. As current passes through the resistor 33a, broad band "noise" is produced that is proportionate in value to the temperature exhibited by the resistor, i.e.,

1/2 KT

where

    K = Boltzman constant

    T = temperature

As illustrated in Figure 7, the broad band noise produced by the resistor 33a is then preferably passed through a series of high band width amplifiers 33b, 33c (e.g., >100MHz) to a high pass filter 33d (e.g., 2-3MHz). The output from the filter 33d is then transmitted to a further amplifier 33f and a low pass filter 33g (e.g., 5-7MHz). The output from the filter 33g is then transmitted to a final high band width amplifier 33h (e.g., >100MHz) and a comparator 33i, which converts the signal to digital noise.

Referring back to Figure 6, the broad band digital noise provided by the noise generator 32 is then preferably passed through a broad band pass filter 34 (e.g., 2.5 - 5.0MHz). The output from the broad band pass filter 34, which is band limited random noise, is then transmitted to a comparator 36.

The output from the comparator 36, which is randomly spaced digital pulses $P_1$-$P_4$ (see figure 8), is then transmitted to the counter-divider 38. The output from the counter-divider 38 is simply a lower frequency set of randomly spaced digital pulses.

As will be appreciated by one having skill in the art, the above described electronic processing means (i.e., "sampling clocking") will provide "true random" sampling that is virtually impervious to artifacts.

To read the spectral waveform that is produced by the sampling means 24 (i. e., random sampling technique) of the invention, an averager is preferably employed. As illustrated in Fig. 9, the averager, which is preferably a sub-system or module of the analyzer 10, includes a reconstruction buffer 40 having a plurality of buckets 42 and a counter 44.

By way of example, assuming the buckets 42 are spaced in time intervals of 200 msec and a first signal is produced at 100 msec after the stimulus, the counter 44 (i.e., synchronous clock) would indicate where the signal sample would be placed (i.e., bucket b1). If the sample were produced during the second interval (e.g., 300 msec), the sample would be placed in the second bucket b2. The noted process would continue through the sampling process. The response signal data or samples in each bucket (e.g., $b_1$ - $b_7$) are then averaged to reconstruct the waveform for the test subject.

Thus, since a synchronous clock is employed to determine where the sample is placed in the buffer 42 and a asynchronous clock determines the sampling points, there will never be synchrony with any fixed frequency artifact.

According to the invention, the noted concept provides a reconstructed waveform that physiologically occurs in time intervals that are representative of the real data (i.e., true response signal), without any extraneous data (i.e., noise signal) that is running at a synchronous rate.

As will be appreciated by one having ordinary skill in the art, the above described spread spectrum technique can be employed with virtually all forms of samples and stimuli since it prevents the stimulus from being in synchronization with external sources and the acquisition of data from being in synchronization with environmental sources (e.g., rf signals).

Without departing from the spirit and scope of this invention, one of ordinary skill can make various changes and modifications to the invention to adapt it to various usages and conditions. As such, these changes and modifications are properly, equitably, and intended to be, within the full range of equivalence of the following claims.

## Claims

1. An audiometric apparatus for testing hearing, comprising:

    stimulus generating means (12) for transmitting at least one stimulus sequence to a subject's inner ear; and
    detection means (22) for detecting the response signal returned from the subject's inner ear in response to said stimulus sequence,
    and **characterised in that** the at least one stimulus sequence is a true random stimulus sequence.

2. The audiometric device of Claim 1, wherein said apparatus includes analyzer means (10) for controlling the stimulus generating means (12) and analyzing said response signal.

3. The audiometric apparatus for testing hearing of Claim 1, wherein:

    said response signal comprises at least a first waveform, said detection means (22) including waveform reconstruction means for reconstructing said first waveform, said reconstruction means including means for applying said at least one true random stimulus sequences to said response signal.

4. The audiometric apparatus of Claim 3, wherein said apparatus includes analyzer means (10) for controlling said detection means (22).

5. The audiometric apparatus of Claim 4, wherein said analyzer means (10) includes means for analyzing said first waveform.

6. The audiometric apparatus of Claim 3

wherein said first waveform comprises a true response signal, and wherein said response signal further comprises a second waveform comprising a noise signal, said reconstruction means further including means for applying said at least one true random stimulus sequence to said first and second waveforms whereby data substantially reflective of said first waveform is acquired.

7. The audiometric apparatus of Claim 1 wherein said response signal comprises at least a first waveform, said detection means (22) including means for applying a plurality of true random frequencies to said response signal to reconstruct said first waveform.

8. The apparatus of Claim 1, wherein said apparatus includes analyzer (10) means for controlling the stimulus generating means (12).

9. The apparatus of Claim 8, wherein said analyzer means (10) includes means for controlling said detection means (22).

10. A method of testing the hearing of a subject, comprising the steps of:

presenting at least one stimulus sequence to said subject's inner ear; and
detecting the response signal returned from the subject's inner ear in response to said stimulus sequence,
and **characterised in that** the at least one stimulus sequence is a true random stimulus sequence.

11. The method of Claim 10, wherein a plurality of said true random stimulus sequences is presented to said subject's ear.

12. The method of Claim 10 said response signal comprises at least one waveform, the method further comprising the steps of:

sampling said response signal waveform by applying a plurality of true random frequencies to said response signal, said sampling providing at least a first set of response signal data;
recording said first set of response signal data; and
reconstructing said response signal waveform from said first set of response signal data.

**Patentansprüche**

1. Ein audiometrischer Apparat für prüfenhörfähigkeit, enthalten: Anregung, die Mittel (12) für das Übermitteln mindestens von einer Anregungreihenfolge dem Innenohr eines Themas erzeugt; und Abfragung Mittel (22) für das Ermitteln des Antwortsignals gingen vom Innenohr des Themas in Erwiderung auf besagte Anregungreihenfolge zurück, und **gekennzeichnet** in dem ist die mindestens eine Anregungreihenfolge eine zutreffende gelegentliche Anregungreihenfolge.

2. Die audiometrische Vorrichtung von Anspruch 1, worin besagter Apparat Analysatormittel (10) für das Steuern der Anregung einschließt, die Mittel (12) erzeugt und besagtes Antwortsignal analysiert.

3. Der audiometrische Apparat für prüfenhörenden Anspruch 1, worin: besagtes Antwortsignal enthält mindestens eine erste Wellenform, besagte Abfragung Mittel (22) einschließlich Wellenformrekonstruktionmittel für das Wieder aufbauen der besagten ersten Wellenform, besagte Rekonstruktionmittel einschließlich Mittel für das Anwenden der besagten mindestens Reihenfolgen mit einen zutreffenden gelegentlichen Anregungen an besagtem Antwortsignal.

4. Der audiometrische Apparat von Anspruch 3, worin besagter Apparat Analysatormittel (10) für steuernde besagte Abfragung Mittel (22) einschließt.

5. Der audiometrische Apparat von Anspruch 4, worin besagte Analysatormittel (10) Mittel für das Analysieren der besagten ersten Wellenform einschließt.

6. Der audiometrische Apparat von Anspruch 3, worin besagte erste Wellenform ein zutreffendes Antwortsignal enthält und worin das besagte Antwortsignal, das weiter ist, eine zweite Wellenform enthält, die ein Störsignal enthält, besagte Rekonstruktionmittel, die weiter Mittel für das Zutreffen gesagt mindestens einer zutreffenden gelegentlichen Anregungreihenfolge auf besagte erste und zweite Wellenformen einschließen, hingegen die Daten, die von besagter erster Wellenform im wesentlichen reflektierend sind, erworben werden.

7. Der audiometrische Apparat von Anspruch 1, worin besagtes Antwortsignal mindestens eine erste Wellenform enthält, besagte Abfragung Mittel (22) einschließlich Mittel für das Anwenden einer Mehrzahl der zutreffenden gelegentlichen Frequenzen an besagtem Antwortsignal, besagte erste Wellenform wieder aufzubauen.

8. Der Apparat von Anspruch 1, worin besagter Apparat Mittel des Analysators (10) für das Steuern der Anregung einschließt, die Mittel (12) erzeugt.

9. Der Apparat von Anspruch 8, worin besagte Analysatormittel (10) Mittel für das Steuern der besagten Abfragung Mittel (22) einschließt.

10. Eine Methode der Prüfung des Hörens von ein Thema, die Schritte von enthalten: Darstellen mindestens von einer Anregungreihenfolge Innenohr des besagten Themas; und das Ermitteln des Antwortsignals ging vom Innenohr des Themas in Erwiderung auf besagte Anregungreihenfolge zurück, und **gekennzeichnet** in dem ist die mindestens eine Anregungreihenfolge eine zutreffende gelegentliche Anregungreihenfolge.

11. Die Methode von Anspruch 10, worin eine Mehrzahl der besagten zutreffenden gelegentlichen Anregungreihenfolgen Ohr des besagten Themas dargestellt wird.

12. Die Methode Anspruch 10 des besagten Antwortsignals enthält mindestens eine Wellenform, das Methode weiter - Enthalten der Schritte von: besagte Antwortsignalwellenform durch das Anwenden einer Mehrzahl der zutreffenden gelegentlichen Frequenzen an besagtem Antwortsignal probieren, besagtes Musterstück, das mindestens einen ersten Satz Antwortsignaldaten bereitstellt; notierender besagter erster Satz Antwortsignaldaten; und Wieder aufbauen der besagten Antwortsignalwellenform von besagtem erstem Satz Antwortsignaldaten.

## Revendications

1. Un appareil audiométrique pour l'audition de essai, comportant : stimulus produisant des moyens (12) de transmettre au moins un ordre de stimulus à l'oreille intérieure d'un sujet ; et le moyen de détection (22) pour détecter le signal de réponse retourné de l'oreille intérieure du sujet en réponse à ledit ordre de stimulus, et **caractérisé en ce que** l'au moins un ordre de stimulus est un véritable ordre aléatoire de stimulus.

2. Le dispositif audiométrique de la revendication 1, où ledit appareil inclut des moyens d'analyseur (10) pour commander le stimulus produisant des moyens (12) et analysant ledit signal de réponse.

3. L'appareil audiométrique pour la revendication entendante parler de essai 1, où : ledit signal de réponse comporte au moins une première forme d'onde, lesdits moyens de détection (22) comprenant des moyens de reconstruction de forme d'onde de reconstruire la première forme d'onde, lesdits moyens de reconstruction comprenant des moyens de s'appliquer au moins ordres aléatoires d'un de véritables stimulus à ledit signal de réponse.

4. L'appareil audiométrique de la revendication 3, où ledit appareil inclut des moyens d'analyseur (10) pour lesdits moyens de contrôle de détection (22).

5. L'appareil audiométrique de la revendication 4, où ledit moyen d'analyseur (10) inclut des moyens d'analyser la première forme d'onde.

6. L'appareil audiométrique de la revendication 3 où ladite première forme d'onde comporte un véritable signal de réponse, et où ledit signal de réponse autre comporte une deuxième forme d'onde comportant un signal de bruit, lesdits moyens de reconstruction incluant plus loin des moyens de s'appliquer dit au moins un véritable ordre aléatoire de stimulus à lesdites premières et deuxièmes formes d'onde par lequel des données essentiellement réfléchissantes de ladite première forme d'onde soient acquises.

7. L'appareil audiométrique de la revendication 1 où ledit signal de réponse comporte au moins une première forme d'onde, lesdits moyens de détection (22) comprenant des moyens de s'appliquer une pluralité de véritables fréquences aléatoires à ledit signal de réponse pour reconstruire ladite première forme d'onde.

8. L'appareil de la revendication 1, où ledit appareil inclut des moyens de l'analyseur (10) de commander le stimulus produisant des moyens (12).

9. L'appareil de la revendication 8, où ledit moyen d'analyseur (10) inclut des moyens de commander lesdits moyens de détection (22).

10. Une méthode d'examiner entendre parler d'un sujet, comportant les étapes de : présentation au moins d'un ordre de stimulus à l'oreille intérieure de ledit sujet ; et détectant le signal de réponse retourné de l'oreille intérieure du sujet en réponse à ledit ordre de stimulus, et **caractérisé en ce que** l'au moins un ordre de stimulus est un véritable ordre aléatoire de stimulus.

11. La méthode de revendication 10, où une pluralité de lesdits véritables ordres aléatoires de stimulus est présentée à l'oreille de ledit sujet.

12. La méthode de signal de réponse de la revendication 10 comporte au moins une forme d'onde, la méthode comportant en outre les étapes de : prélevant ladite forme d'onde de signal de réponse en s'appliquant une pluralité de véritables fréquences aléatoires à ledit signal de réponse, ledit prélèvement fournissant au moins un premier ensemble de données de signal de réponse ; premier ensemble de enregistrement de données de signal de réponse ; et reconstruisant ladite forme d'onde de signal de réponse de ledit premier ensemble de données de signal de réponse.

FIG.-1

FIG.-2

FREQUENCY

X

TIME

*FIG.—3*

*FIG.—4*

*FIG.—6*

FIG.—5

FIG.—7

FREQUENCY

$P_1$  $P_2$  $P_3$  $P_4$

TIME

## *FIG.—8*

40

42 — | $b_1$ | $b_2$ | $b_3$ | $b_4$ | $b_5$ | $b_6$ | $b_7$ |

44 —

$t_0$  $t_1$  $t_2$  $t_3$  $t_4$  $t_5$  $t_6$  $t_7$ ⟶

## *FIG.—9*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4809708 A